# EUROPÄISCHE PATENTSCHRIFT

(11) **EP 2 266 507 B1**
(45) Veröffentlichungstag und Bekanntmachung des Hinweises auf die Patenterteilung: **29.07.2015**
(21) Anmeldenummer: 10157043.0
(22) Anmeldetag: 19.03.2010
(51) Int. Cl.: A61F 2/915

(54) **Stent mit verbessertem Stentdesign**
Stent having improved stent design
Stent ayant un design amélioré

(30) Priorität: 22.06.2009 US 218999 P
(43) Veröffentlichungstag der Anmeldung: 29.12.2010
(73) Patentinhaber: Biotronik VI Patent AG, 6341 Baar (CH)
(72) Erfinder: Mews, Steffen, 18057, Rostock (DE); Bakczewitz, Frank, 18055, Rostock (DE)
(74) Vertreter: Lindner-Vogt, Karin L.

(56) Entgegenhaltungen:
- EP-A2- 1 095 631
- WO-A1-2010/132155
- WO-A2-2006/099449
- US-A1- 2002 123 799
- US-A1- 2004 230 293
- US-A1- 2006 173 530

## Beschreibung

Die Erfindung betrifft einen Stent mit einem verbesserten Stentdesign, insbesondere mit verbessertem Design der Axialverbinder.

### Hintergrund der Erfindung

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu einen Grundkörper auf, der eine Vielzahl von umlaufenden Stützstrukturen z. B. aus metallischen Streben aufweist, der zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind auch Aneurysmenstents bekannt, die eine Stützfunktion für eine beschädigte Gefäßwand bzw. eine Abdichtung von zum Beispiel intrazerebralen Gefäßaussackungen bieten.

Herkömmliche Stents zur Behandlung von Stenosen haben einen zylinderförmigen Grundkörper von ausreichender Tragkraft, der das verengte Gefäß im gewünschten Maße öffnet und offen hält und dadurch den ungehinderten Blutfluss wieder ermöglicht. Die Umfangswandung des Grundkörpers wird in der Regel von einer gitterartigen Tragstruktur gebildet, die es erlaubt, den Stent in einem komprimierten (gecrimpten) Zustand mit kleinem Außendurchmesser bis zur zu behandelnden Engstelle des jeweiligen Gefäßes einzuführen und dort beispielsweise mit Hilfe eines Dilatationsballonkatheters soweit aufzuweiten, dass das Gefäß den gewünschten, vergrößerten Innendurchmesser aufweist. Der Vorgang der Positionierung und Expansion der Stents während der Prozedur und die abschließende Lage des Stents im Gewebe nach Beendigung der Prozedur muss durch den Kardiologen überwacht werden. Dies kann durch bildgebende Verfahren, wie z. B. durch Röntgenuntersuchungen erfolgen.

Der Stent besitzt einen Grundkörper aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßem Gebrauch mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems, in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und biodegradierbare/resorbierbare Werkstoffe unterteilt werden.

Stents weisen einen zylinderförmigen Grundkörper auf, der ein Lumen entlang der axialen Richtung umfasst. Der Grundkörper weist eine Mehrzahl von mäanderförmigen Streben auf, die die umlaufenden Stützstrukturen bilden, z. B. umlaufende zylindrische Mäanderringe oder Helices, die nacheinander folgend entlang der axialen Richtung angeordnet sind. Die Stützstrukturen werden durch Verbindungselemente - sogenannte Axialverbinder oder auch Konnektoren - in axialer Richtung miteinander verbunden. Zumindest bei gefäßstützenden Stents müssen diese Axialverbinder einerseits so angeordnet sein, dass sie eine ausreichende Biegeflexibilität des Stents gewährleisten, andererseits dürfen sie einen Crimp- und/oder Dilatationsprozess nicht behindern.

In US 6,464,720 wird ein Stentdesign vorgeschlagen, bei dem der Stentgrundkörper Öffnungen aufweist. Diese Öffnungen dienen der Aufnahme von radiopaquen Markern aus röntgendichtem Material. Zwar beeinflussen die Öffnungen in diesem Stentdesign die Crimpbarkeit nur minimal, stören aber die homogene plastische Deformation der Stützelemente und haben damit einen wesentlichen negativen Einfluss auf die mechanischen Eigenschaften des Stents.

US 2004/0230293 offenbart einen Stent mit den technischen Merkmalen des Oberbegriffs aus Anspruch 1.

Eine Ursache erhöhter Entzündungsreaktionen im Gefäß nach Stentimplantation ist die gezielt eingesetzte Überdilatation des Stents, die dadurch nötig ist, dass der Stent in einem kurzen Zeitraum nach der Implantation eine gewisse Rückfederung, einen so genannten Recoil, zeigt. Diesen Recoil, dessen Höhe von der jeweiligen Konstruktion und insbesondere vom verwendeten Material abhängig ist, zeigt jede für Implantate verwendete Materialzusammensetzung. Um eine mindeste und physiologisch sinnvolle Lumengröße des behandelten Gefäßes nach Implantation zu erreichen, ist eine Überdilatation des Stents notwendig, um diese Rückfederung auszugleichen. Durch diese Überdilatation wird das Gefäß übermäßig gedehnt; es kommt zur Gefäßschädigung, auf die der Körper mit einer Entzündungsreaktion und einer anschließenden erhöhten Bildung von neuem Gewebe (Neointimaproliferation) reagiert. Beide Reaktionen gilt es, im Rahmen von Stentimplantationen zu mindern.

Gerade bei der Nutzung von Magnesium oder einer Magnesiumlegierung als degradierbares Stentmaterial, welche über nicht besonders günstige mechanische Werkstoffeigenschaften verfügen, sind minimale Beeinflussungen des Kraftflusses in Kombination mit einer effektiven Nutzung des Crimpraumes von besonderer Bedeutung und erfordern somit ein optimales Design der Axialverbinder.

Der vorliegenden Erfindung liegt die Aufgabe zugrunde, die zuvor geschilderten Probleme zu lösen. Insbesondere soll ein Stentdesign bereitgestellt werden, welches eine lediglich minimale Beeinflussung des Kraftflusses in den Stützstreben mit einer effektiven Nutzung des zum Crimpen zur Verfügung stehenden Raumes erlaubt und welches gleichzeitig eine homogene plastische Deformation, insbesondere während der Dilatation des Stentgrundkörpers am Behandlungsort erlaubt. Insbesondere soll der Recoil des Stentkörpers nach der Implantation vermindert werden.

Die Aufgabe wird gelöst durch Bereitstellung eines Stents mit einem eine Zylinderform umschreibenden, aus einer kontrahierten Ausgangsstellung in eine dilatierte Stützstellung radial expandierbaren Grundkörper umfassend eine Vielzahl in Umfangsrichtung umlaufender, in axialer Richtung aneinander gereihter in ihrer Grobstruktur mäanderförmigen Streben aus biegsamem Material, und in axialer Richtung mindestens einen, die mäanderförmigen Streben von zwei in axialer Richtung nebeneinander liegenden Mäanderbögen verbindenden Axialverbinder, wobei der mindestens eine Axialverbinder den Innenradius eines Scheitelpunkts eines ersten Mäanderbogens mit einem zweiten Mäanderbogen verbindet, wobei der mindestens eine Axialverbinder am Innenradius des Scheitelpunkts des ersten Mäanderbogens eine mindestens doppelarmige Struktur aufweist.

Die erfindungsgemäße Lösung zeichnet sich dadurch aus, dass der mindestens eine die mäanderförmigen Streben verbindende Axialverbinder sich mit dem Innenradius des Scheitelpunktes in einer mindestens doppelarmigen Struktur verbindet. Die mindestens doppelarmige Struktur weist drei Arme auf, wobei ein Arm parallel zur axialen Richtung verläuft. Durch diese mindestens doppelarmige Struktur der Verbindung zwischen mäanderförmiger Strebe und Axialverbinder, bleibt eine homogene Verteilung der Spannungen und Dehnungen in den Bogenelementen des Stents unbeeinflusst. Eine homogene plastische Deformierbarkeit der erfindungsgemäßen Stents bleibt so gewährleistet. Durch die mindestens doppelarmige Struktur entstehen zusätzliche plastische Verformungsbereiche im Gesamtsystem des Stents, die zur Versteifung des Systems beitragen.

Dadurch, dass sich die mindestens doppelarmige Struktur des Axialverbinders im Innenradius des Scheitelpunktes anbindet, beansprucht die Anbindung nur wenig Raum, so dass genügend Raum zur Gewährleistung einer ausreichenden Crimpbarkeit und Biegeflexibilität des erfindungsgemäßen Stents zur Verfügung steht.

Dadurch, dass die Anbindungen der mindestens doppelarmigen Struktur des Axialverbinders über den gesamten Innenradius des Scheitelpunktes verteilt sind, weist der erfindungsgemäße Stent eine optimale Kraftübertragung von einer Stützstruktur zur nächsten, sowie die notwendige Stabilität auf. Eine Ausrichtung der Axialverbinder im Wesentlichen entlang der Axialrichtung des Stents führen zu einer optimalen Raumausnutzung im gecrimpten Zustand des erfindungsgemäßen Stents.

Dadurch, dass der erfindungsgemäße Stent eine mindestens doppelarmige Struktur der Axialverbinder aufweist, unterstützen die Axialverbinder den Stent in seiner Standard- und Radialkraft. Die Radialkraft ist diejenige Kraft, die senkrecht auf der Axialrichtung radial nach außen weist und dem Stent die Stützeigenschaften gegenüber dem zu öffnenden Lumen des Blutgefäßes gibt. Durch die Anbindung mittels der mindestens doppelarmigen Struktur entstehen zwischen den Armen der mindestens doppelarmigen Struktur und dem Innenradius des angebundenen Scheitelpunkts Öffnungen. Diese Öffnungen stellen geschlossenen Zellen in der Stentstruktur dar, die die Stentsteifigkeit erhöhen und damit zur Versteifung des Gesamtsystems beitragen. Durch die Versteifung des Gesamtsystems minimiert sich der Recoil des Gesamtsystems. Die Axialverbinder tragen durch die mindestens doppelarmige Struktur zur Erhöhung der Radialkraft und damit der Stützkraft des Stents bei und vermindern den unerwünschten Recoil.

In einer bevorzugten Ausführungsform besteht die mindestens doppelarmige Struktur aus drei Armen, wobei zwei Arme der mindestens doppelarmigen Struktur in der dilatierten Stützstellung, die der Stent nach der Implantation einnimmt, einen Winkel im Bereich von 30° bis 180°, vorzugsweise im Bereich von 60° bis 180°, besonders bevorzugt im Bereich von 90° bis 180° einschließen.

Die mindestens doppelarmige Struktur besteht aus drei Armen. Dabei verläuft der mittlere der drei Arme parallel zur axialen Richtung des Stents. Insgesamt hat die Anordnung der Arme eine Symmetrie in Bezug auf die axiale Richtung. Es sind allerdings auch asymmetrische Ausgestaltungen möglich und umsetzbar. Der Grundkörper des erfindungsgemäßen Stents kann aus jedem Implantatwerkstoff bestehen, der für die Herstellung von Implantaten, insbesondere Stents, geeignet ist. Implantatwerkstoffe für Stents umfassen Polymere, metallische Werkstoffe und keramische Materialien. Biokompatible Metalle und Metalllegierungen für Permanentimplantate beinhalten beispielsweise rostfreie Stähle (zum Beispiel 316L), Kobaltbasislegierungen (zum Beispiel CoCrMo-Gusslegierungen, CoCrMo-Schmiedelegierungen, CoCrWNi-Schmiedelegierungen und CoCrNiMo-Schmiedelegierungen), Reintitan und Titanlegierungen (z. B. cp Titan, TiA16V4 oder TiA16Nb7) und Goldlegierungen. Bevorzugt weist der Grundkörper einen metallischen Implantatwerkstoff auf oder besteht daraus.

Besonders bevorzugt weist der erfindungsgemäße Stent einen Grundkörper auf, der einen biodegradierbaren Implantatwerkstoff enthält oder daraus besteht. Im Bereich biodegradierbarer Stents werden Magnesium oder Reineisen sowie biodegradierbare Basislegierungen der Elemente Magnesium, Eisen, Zink, Molybdän und Wolfram eingesetzt. Insbesondere kann der Grundkörper eines erfindungsgemäßen Stents eine biodegradierbare Magnesiumlegierung aufweisen oder daraus bestehen.

Unter Legierung wird vorliegend ein metallisches Gefüge verstanden, deren Hauptkomponente Magnesium, Eisen, Zink oder Wolfram ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%.

Dabei ist es nicht zwingend notwendig, dass sowohl der Grundkörper als auch der mindestens eine Axialverbinder aus demselben Material bestehen. Es ist im Gegenteil eine beliebige Kombination von Materialien - Metallen und Polymeren - möglich. Bei biodegradierbaren Stents ist darauf zu achten, dass alle verwendeten Materialien biodegradierbar sind.
Abbildungen:

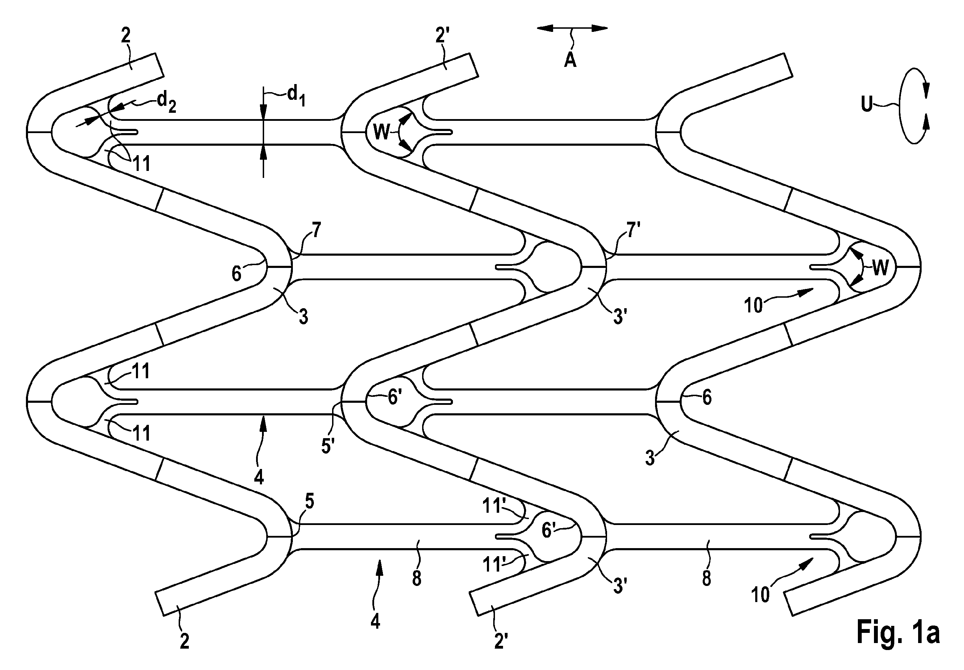

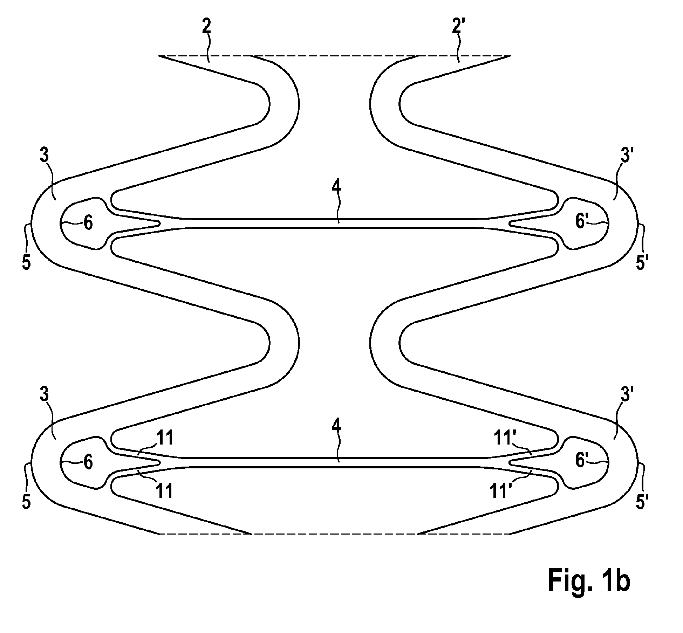

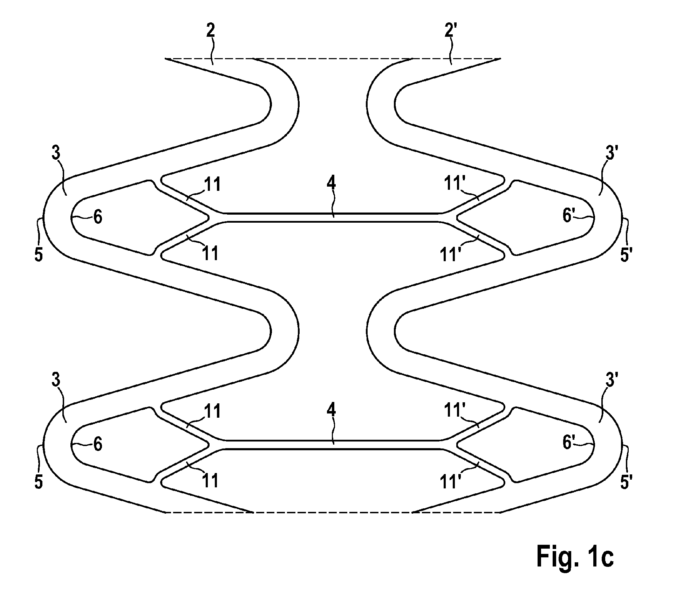

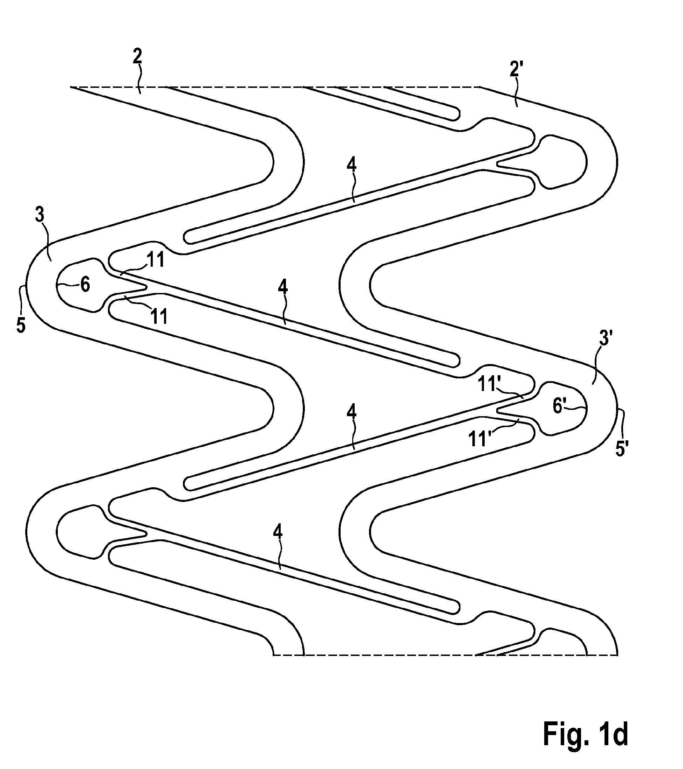

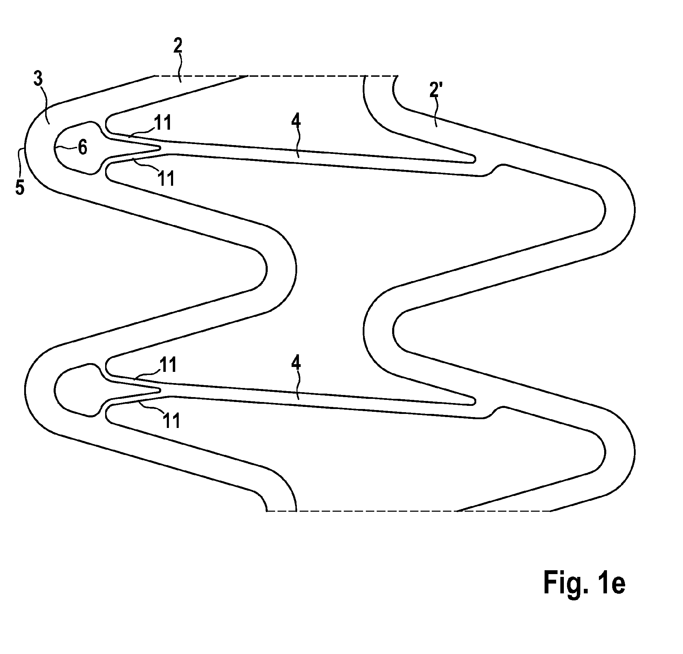

- FIG. 1 a: zeigt schematisch einen Ausschnitt aus einem Grundkörper eines Stents, wobei die mindestens doppelarmige Struktur aus genau zwei Armen besteht, und der Axialverbinder jeweils den Innenradius eines Scheitelpunkts eines ersten Mäanderbogens mit dem Außenradius des Scheitelpunkts eines zweiten Mäanderbogens verbindet,
- FIG. 1 b, c: zeigen schematisch Ausschnitte aus einem Grundkörper eines Stents, wobei die mindestens doppelarmige Struktur aus genau zwei Armen besteht, und der Axialverbinder jeweils den Innenradius eines Scheitelpunkts eines ersten Mäanderbogens mit dem Innenradius des Scheitelpunkts eines zweiten Mäanderbogens verbindet,
- FIG. 1d, e: zeigen schematisch Ausschnitte aus einem Grundkörper eines Stents, wobei die mindestens doppelarmige Struktur aus genau zwei Armen besteht, und der Axialverbinder jeweils den Innenradius eines Scheitelpunkts eines ersten Mäanderbogens mit dem geraden Bereich eines zweiten Mäanderbogens verbindet,
- FIG. 2 a, b: zeigen schematisch Ausschnitte aus einem Grundkörper eines erfindungsgemäßen Stents, wobei die mindestens doppelarmige Struktur aus genau drei Armen besteht,
- FIG. 3: zeigt schematisch einen Ausschnitt aus einem Grundkörper eines erfindungsgemäßen Stents, wobei die mindestens doppelarmige Struktur aus einer Verästelung des Axialverbinders mit fünf Armen besteht.

Die Erfindung wird nachfolgend anhand der Ausführungsbeispiele in Verbindung mit den Abbildungen näher erläutert.

Ein Stent weist einen eine Zylinderform umschreibenden Grundkörper auf, der ein Lumen entlang einer Axialrichtung umschließt. Durch dieses Lumen kann, nach erfolgter Implantation des Stents in ein Blutgefäß, der Blutfluss erfolgen. Der Grundkörper umfasst eine Vielzahl von in Umfangsrichtung umlaufender, in axialer Richtung aneinandergereihter in ihrer Grobstruktur mäanderförmigen Streben, die aus biegsamem Material gebildet sind. Die mäanderförmigen Streben sind im Wesentlichen für die Stützfunktion des Stents verantwortlich. Die notwendige Ausdehnung des Stentgrundkörpers bei Stentimplantation wird durch die Mäanderform der Streben gewährleistet.

Die Mäanderform weist Scheitelpunkte auf, die sich in ihrer Krümmungsrichtung im Verlauf der mäanderförmigen Strebe abwechseln. Auf eine Rechtskrümmung erfolgt nach einem kurzen gradlinigen Verlauf der mäanderförmigen Strebe eine Linkskrümmung. Dieses System setzt sich entlang der Axialrichtung im Wechsel so fort, dass eine ringförmige umlaufende Struktur entsteht, die ein Lumen umschließt. Die Scheitelpunkte weisen einen Innenradius und einen Außenradius auf. Der Innenradius des Scheitelpunkts ist der Bereich, der bei einer gedachten Kreisform des Scheitelpunkts im Inneren des Kreises liegt. Entsprechend ist der Außenradius die Außenbegrenzung der gedachten Kreisform des Scheitelpunktes.

Der Grundkörper umfasst neben einer Vielzahl von Stützstrukturen einen oder mehrere Axialverbinder, wobei zwei aufeinander folgende umlaufende Stützstrukturen über mindestens einen Axialverbinder miteinander verbunden sind. Die Axialverbinder des erfindungsgemäßen Stents sind derart ausgestaltet, dass eine Mehrzahl von Stützstrukturen zu einem Grundkörper verbunden werden kann, der für den Einsatz in einem expandierbaren Stent geeignet ist. Dazu verbindet jeweils ein Axialverbinder einen Scheitelpunkt eines ersten Mäanderbogens einer mäanderförmigen Strebe mit einem zweiten Mäanderbogen einer in Axialrichtung nebenliegenden mäanderförmigen Strebe. Die Scheitelpunkte des ersten und zweiten Mäanderbogens liegen in Axialrichtung parallel bzw. gegenläufig zueinander bzw. versetzt, so dass der Axialverbinder auf der zylinderförmigen Fläche des Grundkörpers entlang läuft. Zwei aufeinander folgende mäanderförmigen Streben können auch über mehr als einen Axialverbinder miteinander verbunden sein. Bevorzugt sind die Axialverbinder nur so lang, dass gerade noch eine ausreichende Flexibilität der beiden benachbarten mäanderförmigen Streben gewährleistet ist, aber nicht so lang, dass der erfindungsgemäße Stent torsionsweich wird. Einer, mehrere oder alle Axialverbinder eines erfindungsgemäßen Stents können eine geschwungene Form aufweisen. Die Axialverbinder sind in grundsätzlich axialer Richtung zwischen den beiden zu verbindenden umlaufenden mäanderförmigen Streben ausgerichtet, wobei die Axialverbinder nicht notwendigerweise in exakt paralleler Ausrichtung zur Axialrichtung vorliegen.

Der erfindungsgemäße Stent weist Axialverbinder auf, die eine langgestreckte Form haben. Der Axialverbinder setzt sich zusammen aus einem Hauptstamm und der mindestens doppelarmigen Struktur. Der Hauptstamm des Axialverbinders geht direkt und unmittelbar in die mindestens doppelarmige Struktur über. Die mindestens doppelarmige Struktur verbindet den Axialverbinder mit dem Innenradius des Scheitelpunktes der mäanderförmigen Strebe. Die mindestens doppelarmige Struktur des Axialverbinders besteht aus mindestens zwei Armen. Die mindestens zwei Arme des Axialverbinders bilden die Verbindung des Hauptstamms mit dem Innenradius des Scheitelpunkts der mäanderförmigen Strebe.

Der Hauptstamm des Axialverbinders weist eine Stegbreite d₁ und die Arme der mindestens doppelarmigen Struktur weisen eine Stegbreite d₂ auf. Dabei ist d₁ größer als d₂. Die Durchmesser der Arme können jedoch auch verschieden groß sein.

An seinen beiden Enden weist der Axialverbinder Verbindungsstellen zu der mäanderförmigen Strebe auf. An den Verbindungsstellen der mindestens doppelarmigen Struktur gehen die mindestens zwei Arme über in den Innenradius des Scheitelpunktes der mäanderförmigen Struktur. Dabei kann der Anbindungsort der Arme auch auf dem geraden Teil des Mäanderbogens liegen.

Die Anbindung des mindestens einen Axialverbinder an dem zweiten Mäanderbogen wird realisiert entweder mittels einer Anbindung an einen Außenradius eines Scheitelpunktes des zweiten Mäanderbogens, an einen Innenradius eines Scheitelpunktes des zweiten Mäanderbogens oder an einen Punkt auf der gradlinigen Verbindung zwischen dem Innenradius und dem Außenradius des zweiten Mäanderbogens ("Strut"). Die Anbindung des mindestens einen Axialverbinder an dem zweiten Mäanderbogen kann wiederum durch eine mehrarmige Struktur erfolgen.

Die Stegbreite der mindestens zwei Arme der doppelarmigen Struktur und die Stegbreite des Hauptstammes an der Verbindungsstelle unmittelbar vor dem Übergang in die mäanderförmige Struktur sind größer als die jeweiligen Stegbreiten d₁ bzw. d₂. Die Stegbreite der Verbindungsstellen verjüngt sich kontinuierlich in Richtung der Axialverbinder. Diese Verjüngung kann gleichmäßig erfolgen. Die Verjüngung kann aber auch ungleichmäßig erfolgen.

In FIG. 1a ist ein Ausschnitt aus einem Grundkörper eines Stents gezeigt. Dargestellt sind Abschnitte von zwei in Umfangsrichtung U umlaufenden, in axialer Richtung A aneinandergereihter mäanderförmigen Streben 2 und 2', die über Axialverbinder 4 miteinander verbunden sind. Die mäanderförmigen Streben 2 und 2' weisen Mäanderbögen 3 und 3' auf. Die Mäanderbögen 3 und 3' haben Scheitelpunkte 5 und 5', die einen Innenradius 6, 6' und einen Außenradius 7, 7' aufweisen. Die Axialverbinder 4 verbinden den Innenradius 6 des Scheitelpunkts 5 mit dem Außenradius 7' des Scheitelpunkts 5' der in axialer Richtung A nächstliegenden mäanderförmigen Strebe 2'. Die mindestens doppelarmige Struktur wird in FIG. 1a durch jeweils zwei Arme 11 und 11' realisiert.

Der Axialverbinder 4 setzt sich zusammen aus einem Hauptstamm 8 und der doppelarmigen Struktur 10. Der Hauptstamm 8 des Axialverbinders geht direkt und unmittelbar in die doppelarmige Struktur 10 über. Der Hauptstamm 8 des Axialverbinders weist eine Stegbreite d₁ und die zwei Arme 11 bzw. 11' der doppelarmigen Struktur 10 weisen eine Stegbreite d₂ auf. Dabei ist d₁ größer als d₂.

Bei der Ausführungsform mit einer doppelarmigen Struktur beschreiben die beiden Arme zusammen mit dem Innenradius des Scheitelpunkts des Mäanderbogens eine Öffnung, die eine kellenartige Form aufweist.

In den FIG. 1b und 1c sind Ausschnitte aus einem Grundkörper eines Stents gezeigt. Dargestellt sind Abschnitte von zwei in Umfangsrichtung U umlaufenden, in axialer Richtung A aneinandergereihter mäanderförmigen Streben 2 und 2', die über Axialverbinder 4 miteinander verbunden sind. Die mäanderförmigen Streben 2 und 2' weisen Mäanderbögen 3 und 3' auf. Die Mäanderbögen 3 und 3' haben Scheitelpunkte 5 und 5', die einen Innenradius 6 und einen Außenradius 7 aufweisen. Dargestellt sind zwei verschiedenen Varianten einer so genannten Valley-to-valley-Anbindung; das heißt, dass die Axialverbinder 4 den Innenradius 6 des Scheitelpunkts 5 mit dem Innenradius 6' des Scheitelpunkts 5' der in axialer Richtung A nächstliegenden mäanderförmigen Strebe 2' verbinden. Die mindestens doppelarmige Struktur wird in den FIG. 1b und c durch zwei Arme 11 und zwei Arme 11' realisiert.

In den FIG. 1d und 1e sind Ausschnitte aus einem Grundkörper eines Stents gezeigt. Dargestellt sind Abschnitte von zwei in Umfangsrichtung U umlaufenden, in axialer Richtung A aneinandergereihter mäanderförmigen Streben 2 und 2', die über Axialverbinder 4 miteinander verbunden sind. Die mäanderförmigen Streben 2 und 2' weisen Mäanderbögen 3 und 3' auf. Die Mäanderbögen 3 und 3' haben Scheitelpunkte 5 und 5', die einen Innenradius 6 und einen Außenradius 7 aufweisen. Dargestellt sind zwei verschiedenen Varianten einer so genannten Valley-to-Strut-Anbindung; das heißt, dass die Axialverbinder 4 den Innenradius 6 des Scheitelpunkts 5 mit dem geraden Bereich der in axialer Richtung A nächstliegenden mäanderförmigen Strebe 2' verbinden. Die mindestens doppelarmige Struktur wird in FIG. 1d und e durch zwei Arme 11 und zwei Arme 11' realisiert.

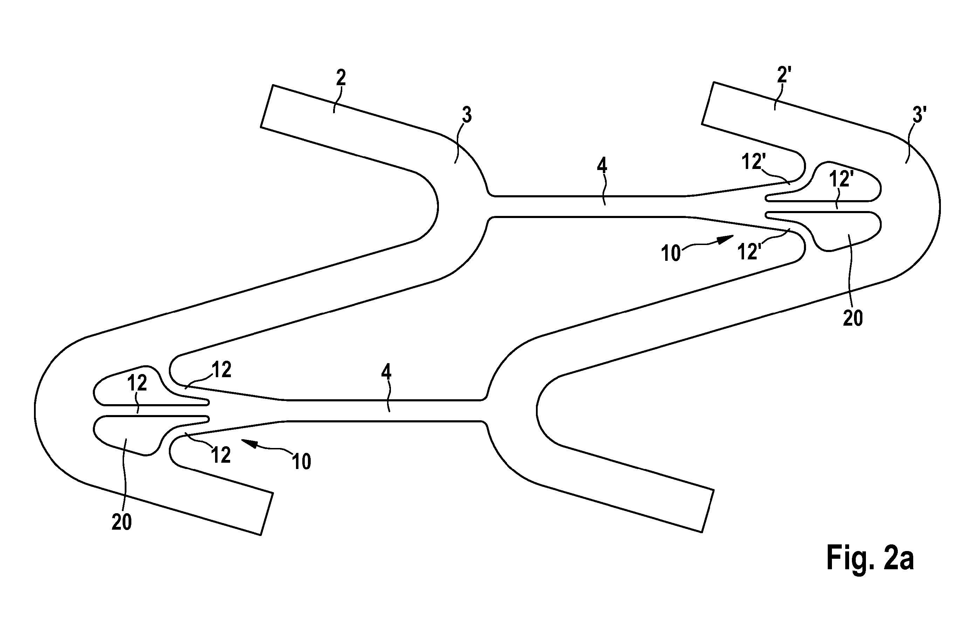

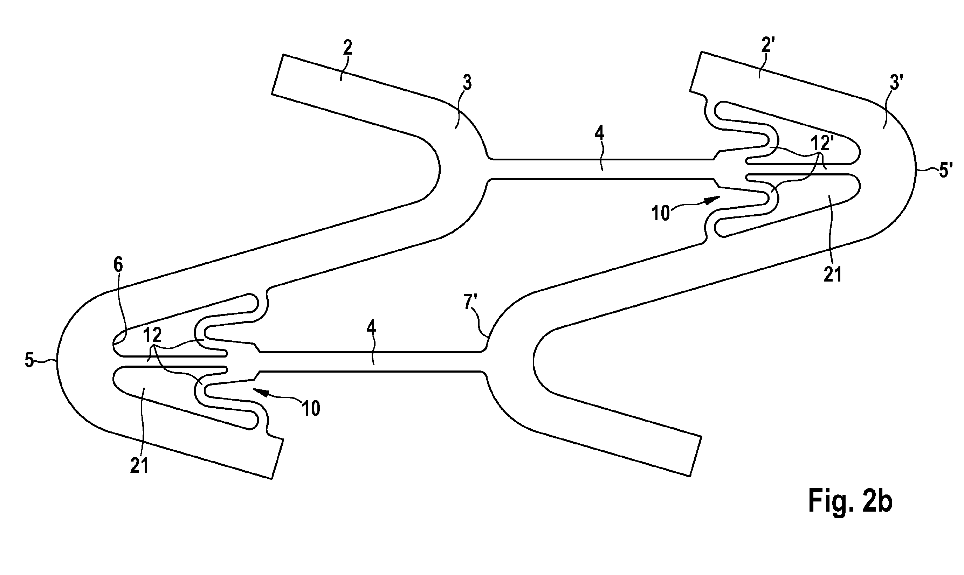

In den FIG. 2a und 2b sind Ausschnitte aus einem Grundkörper eines erfindungsgemäßen Stents gezeigt. Dargestellt sind Abschnitte von zwei in Umfangsrichtung U umlaufenden, in axialer Richtung A aneinandergereihter mäanderförmigen Streben 2 und 2', die über Axialverbinder 4 miteinander verbunden sind. Die mäanderförmigen Streben 2 und 2' weisen Mäanderbögen 3 und 3' auf. Die Mäanderbögen 3 und 3' haben Scheitelpunkte 5 und 5', die einen Innenradius 6 und einen Außenradius 7 aufweisen. Die Axialverbinder 4 verbinden den Innenradius 6 des Scheitelpunkts 5 mit dem Außenradius 7 des Scheitelpunkts 5' der in axialer Richtung A nächstliegenden mäanderförmigen Strebe 2'. Die mindestens doppelarmige Struktur wird in den FIG. 2a und 2b durch drei Arme 12 bzw. 12' realisiert, so dass eine dreiarmige Struktur entsteht. Der Axialverbinder 4 setzt sich zusammen aus einem Hauptstamm 8 und der dreiarmigen Struktur 10. Der Hauptstamm 8 des Axialverbinders geht direkt und unmittelbar in die dreiarmige Struktur 10 über. Es ist ein Anbindungsdesign mit kellenförmigen Öffnungen 20 (FIG. 2a) und ein Design, bei dem die Öffnungen, die die Arme 12 bzw. 12' mit der mäanderförmigen Strebe 2 bzw. 2' einschließen, eine kronleuchterähnliche Form 21 aufweisen (FIG. 2b).

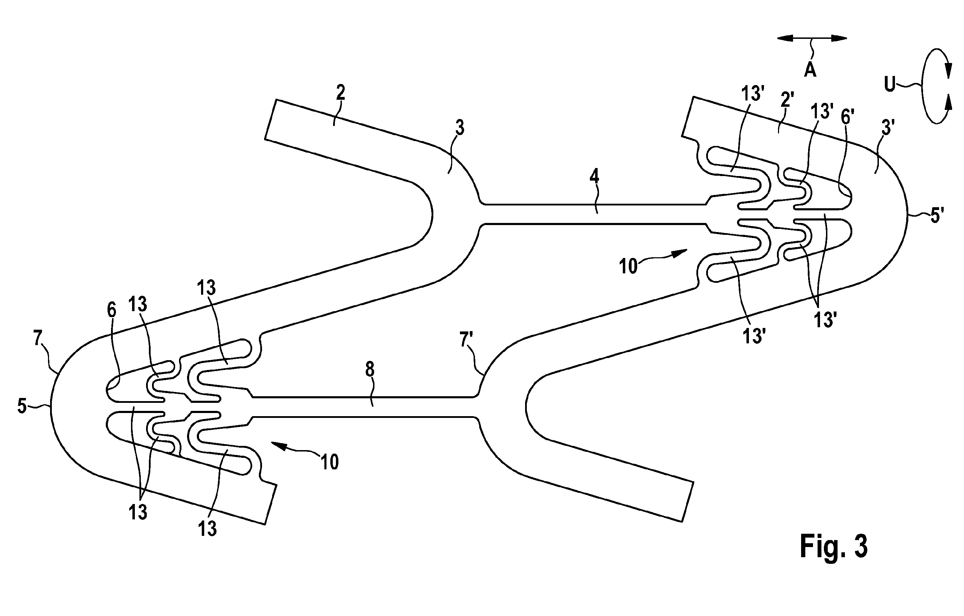

In FIG. 3 ist ein Ausschnitt aus einem Grundkörper eines erfindungsgemäßen Stents gezeigt. Dargestellt sind Abschnitte von zwei in Umfangsrichtung U umlaufenden, in axialer Richtung A aneinandergereihter mäanderförmigen Streben 2 und 2', die über Axialverbinder 4 miteinander verbunden sind. Die mäanderförmigen Streben 2 und 2' weisen Mäanderbögen 3 und 3' auf. Die Mäanderbögen 3 und 3' haben Scheitelpunkte 5 und 5', die einen Innenradius 6 und einen Außenradius 7, 7' aufweisen. Die Axialverbinder 4 verbinden den Innenradius 6 des Scheitelpunkts 5 mit dem Außenradius 7' des Scheitelpunkts 5' der in axialer Richtung A nächstliegenden mäanderförmigen Strebe 2'. Die mindestens doppelarmige Struktur wird in FIG. 3 durch fünf Arme 13, 13' realisiert. Der Axialverbinder 4 setzt sich zusammen aus einem Hauptstamm 8 und der Verästelung der mindestens doppelarmigen Struktur 10. Der Hauptstamm 8 des Axialverbinders geht direkt und unmittelbar in die fünf Arme 13 bzw. 13' der mindestens doppelarmigen Struktur 10 über.

Bei der Ausführungsform mit einer fünfarmigen Struktur beschreiben die fünf Arme 13 bzw. 13' zusammen mit dem Innenradius 6 bzw. 6' des Scheitelpunkts des Mäanderbogens jeweils eine Öffnung, die eine kronleuchterähnliche Form aufweist.

Neben nicht-degradierbaren metallischen Legierungen können auch degradierbare Metalle sowie deren Legierungen für die Umsetzung der Erfindung verwendet werden. Die Legierungen der Elemente Magnesium, Eisen, Zink oder Wolfram sind so in ihrer Zusammensetzung gewählt, dass sie biodegradierbar sind. Als biodegradierbar im Sinne der Erfindung werden Legierungen bezeichnet, bei denen in physiologischer Umgebung ein Abbau stattfindet, der letztendlich dazu führt, dass das gesamte Implantat oder der aus dem Werkstoff gebildete Teil des Implantates seine mechanische Integrität verliert. Als Prüfmedium zur Testung des Degradationsverhaltens einer in Frage kommenden Legierung dient künstliches Plasma, wie es nach EN ISO 10993-15:2000 für Biodegradationsuntersuchungen vorgeschrieben ist (Zusammensetzung NaCl 6,8 g/l, CaCl₂ 0,2 g/l, KCl 0,4 g/l, MgSO₄ 0,1 g/l, NaHCO₃ 2,2 g/l, Na₂HPO₄ 0,126 g/l, NaH₂PO₄ 0,026 g/l). Eine Probe der zu untersuchenden Legierung wird dazu in einem verschlossenen Probenbehälter mit einer definierten Menge des Prüfmediums bei 37 °C gelagert. In zeitlichen Abständen - abgestimmt auf das zu erwartende Degradationsverhalten - von wenigen Stunden bis zu mehreren Monaten werden die Proben entnommen und in bekannter Weise auf Degradationsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Der Begriff Degradation bezieht sich vorliegend auf die Reaktion eines metallischen Werkstoffes mit seiner Umgebung, wobei eine messbare Veränderung des Werkstoffs bewirkt wird, die - bei Einsatz des Werkstoffs in einem Bauteil - zu einer Beeinträchtigung der Funktion des Bauteils führt. Ein Degradationssystem besteht vorliegend aus dem degradierenden metallischen Werkstoff sowie einem flüssigen Degradationsmedium, das in seiner Zusammensetzung die Verhältnisse in physiologischer Umgebung nachstellt oder ein physiologisches Medium, insbesondere Blut ist. Werkstoffseitig beeinflussen die Degradation Faktoren wie die Zusammensetzung und Vorbehandlung der Legierung, mikro- und submikroskopische Inhomogenitäten, Randzoneneigenschaften, Temperatur- und Spannungszustand und insbesondere die Zusammensetzung einer die Oberfläche bedeckenden Schicht. Auf Seiten des Mediums wird der Degradationsprozess durch Leitfähigkeit, Temperatur, Temperaturgradienten, Azidität, Volumen-Oberflächen-Verhältnis, Konzentrationsunterschied sowie Strömungsgeschwindigkeit beeinflusst.

In DE 197 31 021 A1 werden geeignete biodegradierbare metallische Implantatwerkstoffe vorgestellt, deren Hauptbestandteil ein Element aus der Gruppe Alkalimetalle, Erdalkalimetalle, Eisen, Zink und Aluminium ist. Als besonders geeignet werden Legierungen auf Basis von Magnesium, Eisen und Zink beschrieben. Nebenbestandteile der Legierungen können Mangan, Kobalt, Nickel, Chrom, Kupfer, Kadmium, Blei, Zinn, Thorium, Zirkonium, Silber, Gold, Palladium, Platin, Silizium, Kalzium, Lithium, Aluminium, Zink und Eisen sein. Weiterhin ist aus der DE 102 53 634 A1 der Einsatz einer biodegradierbaren Magnesiumlegierung mit einem Anteil von Magnesium >90%, Yttrium 3,7 - 5,5%, Seltenerdmetallen 1,5 - 4,4% und Rest < 1% bekannt, die sich insbesondere zur Herstellung einer Endoprothese, z. B. in Form eines selbstexpandierenden oder ballonexpandierbaren Stent, eignet.

### Bezugszeichenliste:

- A: Axialrichtung
- U: Umfangsrichtung

- 2: mäanderförmige Strebe
- 3, 3': Mäanderbögen
- 4: Axialverbinder
- 5, 5': Scheitelpunkt
- 6, 6': Innenradius des Scheitelpunkts
- 7, 7': Außenradius des Scheitelpunkts
- 8: Hauptstamm des Axialverbinders 4

- 10: mindestens doppelarmige Struktur
- 11, 11': Arme der mindestens doppelarmigen Struktur 10
- 12, 12': Arme der mindestens doppelarmigen Struktur 10 bei drei Armen
- 13: Arme der mindestens doppelarmigen Struktur 10 bei fünf Armen

- 20: kellenförmige Öffnung
- 21: kronleuchterförmige Öffnung

## Patentansprüche

1. Stent mit einem eine Zylinderform umschreibenden, aus einer kontrahierten Ausgangsstellung in eine dilatierte Stützstellung radial expandierbaren Grundkörper umfassend
- eine Vielzahl in Umfangsrichtung (U) umlaufender, in axialer Richtung (A) aneinander gereihter in ihrer Grobstruktur mäanderförmigen Streben (2) aus biegsamem Material, und
- in axialer Richtung (A) mindestens einen, die mäanderförmigen Streben (2) von zwei in axialer Richtung nebeneinander liegenden Mäanderbögen (3, 3') verbindenden Axialverbinder (4), wobei der mindestens eine Axialverbinder (4) den Innenradius (6) eines Scheitelpunkts (5) eines ersten Mäanderbogens (3) mit einem zweiten Mäanderbogen (3') verbindet, und wobei
der mindestens eine Axialverbinder (4) am Innenradius (6) des Scheitelpunkts (5) des ersten Mäanderbogens (3) eine mindestens doppelarmige Struktur (10) aufweist, **dadurch gekennzeichnet, dass** die mindestens doppelarmige Struktur (10) drei Arme (12, 12') aufweist, wobei ein Arm parallel zur axialen Richtung verläuft.

2. Stent nach Anspruch 1, **dadurch gekennzeichnet, dass** die mindestens doppelarmige Struktur (10) zwei (11 bzw 11') der drei Arme aufweist,
die in der dilatierten Stützstellung einen Winkel im Bereich von 30° bis 180°, vorzugsweise im Bereich von 60° bis 180°, besonders bevorzugt im Bereich von 90° bis 180° einschließen.

3. Stent nach Anspruch 1, **dadurch gekennzeichnet, dass** der mittlere Arm der drei Arme (12, 12') der mindestens doppelarmigen Struktur (10) parallel zur axialen Richtung verläuft.

4. Stent nach einem der vorhergehenden Ansprüche, **dadurch gekennzeichnet, dass** das biegsame Material ein Material ausgewählt aus der Gruppe bestehend aus Metallen, Metalllegierungen und/oder Polymeren ist.

5. Stent nach einem der vorhergehenden Ansprüche, **dadurch gekennzeichnet, dass** das biegsame Material ein biodegradierbares Material ist.

6. Stent nach Anspruch 5, **dadurch gekennzeichnet, dass** das biodegradierbare Material ein Material ist ausgewählt aus der Gruppe bestehend aus Magnesium, Eisen, Zink, Wolfram und/oder einer Metalllegierungen aus einem der vorbenannten Metalle.

## Claims

1. A stent comprising a base body, which circumscribes a cylindrical shape and is radially expandable from a contracted starting position into a dilated support position, having
- a plurality of struts (2), which have a basic serpentine structure, are made from a flexible material, extend in the circumferential direction (U), and are arranged in a row in the axial direction (A), and,
- in the axial direction (A), said base body comprises at least one axial connector (4), which connects the serpentine struts (2) of two meander curves (3, 3') arranged next to one another in the axial direction, wherein the at least one axial connector (4) connects the inner radius (6) of a summit (5) of a first meander curve (3) to a second meander curve (3'), and wherein
the at least one axial connector (4) has an at least double-arm structure (10) on the inner radius (6) of the summit (5) of the first meander curve (3), **characterized in that** the at least double-arm structure (10) comprises three arms (12, 12'), wherein one arm extends parallel to the axial direction.

2. The stent according to claim 1, **characterized in that** the at least double-arm structure (10) comprises two (11 and 11') of the three arms, which, in the dilated support position, enclose an angle in the range of 30° to 180°, preferably in the range of 60° to 180°, particularly preferably in the range of 90° to 180°.

3. The stent according to claim 1, **characterized in that** the middle arm of the three arms (12, 12') of the at least double-arm structure (10) extends parallel to the axial direction.

4. The stent according to any one of the preceding claims, **characterized in that** the flexible material is a material selected from the group consisting of metals, metal alloys, and/or polymers.

5. The stent according to one of the preceding claims, **characterized in that** the flexible material is a biodegradable material.

6. The stent according to claim 5, **characterized in that** the biodegradable material is a material selected from the group consisting of magnesium, iron, zinc, tungsten, and/or a metal alloy of one of the aforementioned metals.

## Revendications

1. Endoprothèse vasculaire avec un corps de base, décrivant une forme cylindrique, et se dilatant radialement vers une position de maintien dilatée à partir d'une position de départ contractée, comprenant
- une multiplicité de traverses (2), en forme de méandres parcourant la direction circonférentielle (U), alignées les unes par rapport aux autres dans leur structure globale dans la direction axiale (A), constituées d'un matériau flexible, et
- en direction axiale (A), au moins une connexion axiale (4) reliant les traverses (2) en forme de méandres de deux arcs de méandre (3, 3') situés l'un à côté de l'autre dans la direction axiale, où l'au moins une connexion axiale (4) relie le rayon interne (6) du sommet (5) d'un premier arc de méandre (3) avec un deuxième arc de méandre (3'), et où
l'au moins une connexion axiale (4) présente une structure (10) à au moins deux branches sur le rayon interne (6) du sommet (5) du premier arc de méandre (3), **caractérisé en ce que** la structure (10) à au moins deux branches présente trois branches (12, 12'), dans laquelle une branche s'étend parallèlement par rapport à la direction axiale.

2. Endoprothèse vasculaire selon la revendication 1, **caractérisé en ce que** la structure (10) à au moins deux branches présente deux (11, respectivement, 11') des trois branches qui font un angle dans un domaine entre 30° et 180°, de préférence dans un domaine entre 60° et 180°, de manière particulièrement préférée, dans le domaine entre 90° et 180°, dans la position de maintien dilatée.

3. Endoprothèse vasculaire selon la revendication 1, **caractérisé en ce que** la branche centrale parmi les trois branches (12, 12') de la structure (10) à au moins deux branches s'étend parallèlement par rapport à la direction axiale.

4. Endoprothèse vasculaire selon l'une des revendications précédentes, **caractérisé en ce que** le matériau flexible est choisi dans le groupe constitué par des métaux, des alliages métalliques et/ou des polymères.

5. Endoprothèse vasculaire selon l'une des revendications précédentes, **caractérisé en ce que** le matériau flexible est un matériau biodégradable.

6. Endoprothèse vasculaire selon la revendication 5, **caractérisé en ce que** le matériau biodégradable est un matériau choisi dans le groupe constitué par le magnésium, le fer, le zinc, le tungstène et/ou des alliages métalliques constitués par un des métaux cités précédemment.
